(19) Europäisches Patentamt
European Patent Office
Office européen des brevets

(11) Numéro de publication: **0 044 262**
**B1**

(12) # FASCICULE DE BREVET EUROPEEN

(45) Date de publication du fascicule du brevet: **23.11.83**

(51) Int. Cl.³: **C 07 D 211/90**, C 07 D 213/82, C 07 D 213/85, A 01 N 43/40

(21) Numéro de dépôt: **81420106.7**

(22) Date de dépôt: **16.07.81**

(54) **Nouveaux dérivés de l'aniline, leur préparation et leur application pour le désherbage sélectif de cultures.**

(30) Priorité: **16.07.80 FR 8015993**
**16.07.80 FR 8015994**

(43) Date de publication de la demande:
**20.01.82 Bulletin 82/3**

(45) Mention de la délivrance du brevet:
**23.11.83 Bulletin 83/47**

(84) Etats contractants désignés:
**AT BE CH DE FR GB IT LI LU NL SE**

(56) Documents cités:
**EP - A - 0 003 105**
**FR - A - 2 248 028**
**NL - C - 77 955**

**PATENT ABSTRACTS OF JAPAN, vol. 1, no. 70, 08-07-1977**

(73) Titulaire: **RHONE-POULENC AGROCHIMIE, 14-20, rue Pierre Baizet, F-69009 Lyon (FR)**

(72) Inventeur: **de Reinach Hirtzbach, François, 107 rue Garibaldi, F-69006 Lyon (FR)**
Inventeur: **Ambrosi, Dominique, 15 Allée des Hautinières, F-69260 Charbonnieres les Bains (FR)**

(74) Mandataire: **Chaumette, Michel et al, RHONE-POULENC AGROCHIMIE BP 9163-09, F-69263 Lyon Cedex 1 (FR)**

**EP 0 044 262 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

# Nouveaux dérivés de l'aniline, leur préparation et leur application pour le désherbages sélectif de cultures

La présente invention concerne de nouveaux dérivés de l'aniline, leur préparation, les compositions herbicides les contenant ainsi que leur application pour le désherbage sélectif de cultures, notamment du coton et du tournesol.

L'invention concerne les nouveaux dérivés de l'aniline répondant à la formule:

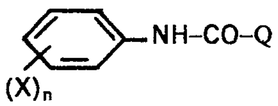

(I)

dans laquelle les substituants ont les significations indiquées ci-après, étant entendu que, dans ce qui suit, sauf indication contraire, l'adjectif «inférieur», appliqué à un radical organique signifie que ce radical comporte au plus six atomes de carbone.

Dans la formule I:

– X représente un atome ou radical choisi parmi:

- les atomes d'halogènes,
- les radicaux alkyle inférieurs,
- les radicaux alkoxy inférieurs,
- les radicaux alcényle inférieurs,
- les radicaux alcényloxy inférieurs,
- les radicaux alkyle inférieurs substitués par un ou plusieurs halogènes,
- le radical amino éventuellement substitué par un ou deux radicaux alkyle inférieurs,

identiques ou différents, ou par le radical $-CO-R_1$ dans lequel $R_1$ représente un radical alkyle inférieur, alkoxy inférieur, alkylamino inférieur ou dialkylamino dans lequel chacune des parties alkyle inférieurs peuvent être identiques ou différentes,

- le radical nitro,
- et le radical cyano.

– n est un nombre entier de 0 à 5 étant entendu que lorsque n est supérieur à 1, les substituants X peuvent être identiques ou différents.

– Q représente un radical répondant à l'une des formules (IA) et (IB) ci-après:

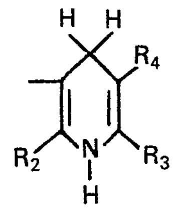

(IA)

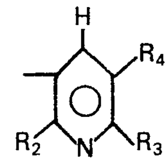

(IB)

dans lesquelles:

– $R_2$, $R_3$, identiques ou différentes, représentent chacun un atome d'hydrogène ou un radical alkyle inférieur,

– $R_4$ représente le radical cyano ou un radical $-COR_5$ dans lequel $R_5$ représente un hydroxy ou un radical $-OR_6$ dans lequel $R_6$ représente un radical alkyle inférieur.

L'invention concerne, de plus, les sels acceptables en agriculture des composés selon la formule (I), notamment les sels formés par les composés selon la formule (I) pour lesquels $R_4$ représente le radical $-COOH$, avec une base minérale ou organique (par exemple les sels de potassium, les sels d'ammonium, les sels d'amines primaires, secondaires ou tertiaires, éventuellement substituées par un ou plusieurs radicaux alcanol), et d'autre part les sels formés avec un acide minéral ou organique par les composés selon la formule (I) pour lesquels Q représente le radical (IB) (par exemple les chlorhydrates de ces composés).

L'invention concerne également les formes tautomères du composé selon la formule I pour lequel le radical Q représente le motif dihydropyridine répondant à la formule (IA).

Certains dérivés de la dihydro-1,4-N-phénylcarbamoyl-3-pyridine ont déjà été décrits dans la littérature, ainsi la demande de brevet français No 2 248 028 décrit en tant que médicament le composé de formule:

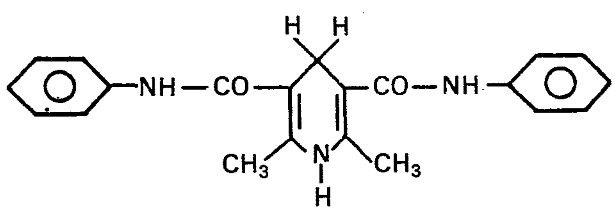

(II)

Ce composé ne présente toutefois pas le niveau d'activité nécessaire pour une utilisation comme herbicide en agriculture.

Certains dérivés de N-phénylcarbamoyl-pyridine ont déjà été décrits dans la littérature: ainsi la demande de brevet européen No 0003105 décrit la préparation de la diméthyl-2,6 dicarboxanilide-3,5 pyridine et indique que ce type de composé est utilisable comme agent de protection des plantes. Toutefois, ce composé ne présente pas le niveau d'activité nécessaire pour une utilisation comme herbicide en agriculture.

Les composés selon l'invention sont différents de ceux décrits dans les demandes de brevets citées plus haut. Ils présentent généralement une excellente activité herbicide.

Parmi les composés répondant à la formule (I), l'invention concerne plus particulièrement ceux pour lesquels:

– $R_2$ et $R_3$, identiques ou différents, représentent l'atome d'hydrogène ou un radical alkyle contenant de 1 à 3 atomes de carbone,

– $R_4$ représente un radical alkoxycarbonyle

comportant de 2 à 6 atomes de carbone ou le radical cyano,

– X représente un atome ou radical choisi parmi les atomes d'halogènes, les radicaux alkyle comportant de 1 à 4 atomes de carbone, halogénoalkyle comportant de 1 à 4 atomes de carbone et un ou plusieurs halogènes, cyano, alkényle comportant de 3 à 5 atomes de carbone,

– n est un nombre entier pouvant être égal à 0, 1, 2, 3 ou 4, étant entendu que lorsque n est supérieur à 1, les substituants X peuvent être soit identiques, soit différents.

Parmi ces composés, une sous famille préférée du fait de son excellente activité herbicide est constituée par les composés répondant à la formule (III):

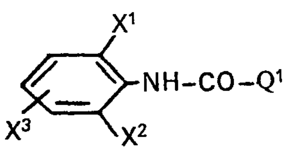

(III)

dans laquelle:

– $X_1$ représente un radical alkyle comportant de 1 à 3 atomes de carbone (de préférence méthyle ou éthyle),

– $X_2$ représente l'atome d'hydrogène ou un radical alkyle comportant de 1 à 3 atomes de carbone (de préférence méthyle ou éthyle),

– $X_3$ représente un atome d'hydrogène ou d'halogène (de préférence chlore) ou un radical alkyle comportant de 1 à 3 atomes de carbone,

– $Q_1$ représente un radical répondant à l'une des formules (IIIA) et (IIIB) ci-après:

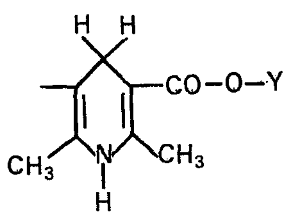

(IIIA)

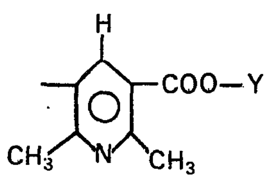

(IIIB)

dans lesquelles:

– Y représente un radical alkyle comportant de 1 à 4 atomes de carbone.

Parmi les composés préférés répondant à la formule (III), on peut citer tout particulièrement les composés suivants:

dihydro-1,4 N-(diéthyl)-2,6 phényl)-carbamoyl-3-éthoxy-carbonyl-5 lutidine-2,6,

– dihydro-1,4 N-(diméthyl-2,6-phényl)-carbamoyl-3 éthoxycarbonyl-5 lutidine-2,6,

– dihydro-1,4 N-(diéthyl-2,6-phényl)-carbamoyl-3 méthoxycarbonyl-5 lutidine-2,6,

– dihydro-1,4 N-(éthyl-2 méthyl-6 phényl)-carbamoyl-3 éthoxycarbonyl-5 (lutidine-2,6.

– dihydro-1,4 N-(chloro-3 diméthyl-2,6 phényl)- carbamoyl-3 méthoxycarbonyl-5 lutidine-2,6.

– diméthyl-2,6 N-(diméthyl-2,6 phényl)-carbamoyl-3 éthoxycarbonyl-5 pyridine.

– diméthyl-2,6 N-(diéthyl-2,6-phényl)-carbamoyl-3 éthoxycarbonyl-5 pyridine.

– diméthyl-2,6 N- (chloro-3 diméthyl-2,6-phényl) -carbamoyl-3 éthoxycarbonyl-5 pyridine.

Les composés selon la formule (I) peuvent être préparés selon un procédé qui consiste à faire réagir ensemble l'anilide de formule (IV), le formaldéhyde de formuel (V) et le dérivé amino-éthylénique de formule (VI).

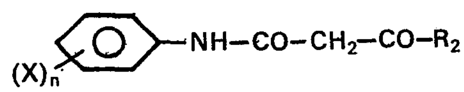

(IV)

H–CH=O (V)

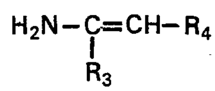

(VI)

dans lesquelles X, n, $R_2$, $R_3$ et $R_4$ ont la même signification que dans les formules (I) et (IA), selon le schéma réactionnel:

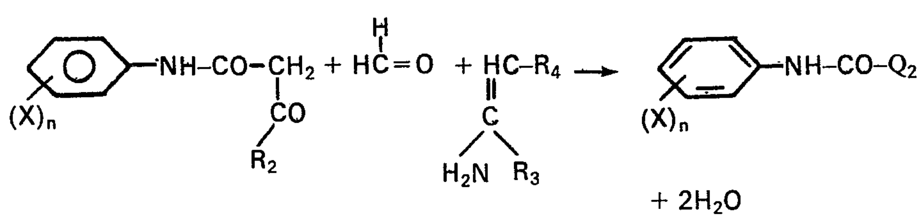

(VII)

dans lequel $Q_2$ représente le radical (IA) dans lequel $R_2$, $R_3$ et $R_4$ ont la même signification que précédemment, puis, si désiré, à déshydrogéner le radical $Q_2$ du composé (VII) résultant de l'étape précédente, pour donner le composé de formule:

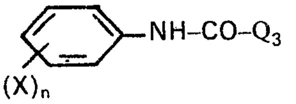

(VIII)

dans lequel X et n sont définis comme précédemment et $Q_3$ représente le radical (IB) dans lequel $R_2$, $R_3$ et $R_4$ ont même signification que précédemment.

La réaction de préparation du composé (VII) est exothermique. Elle s'effectue en milieu solvant organique dès la température ambiante. Elle peut également s'effectuer à température plus élevée (étant entendu que cette température doit rester

inférieure à la température de dégradation thermique des réactifs de départ et des produits formés. Des températures comprises entre 15 °C et 100 °C donnent généralement de bons résultats.

Comme solvants appropriés pour la préparation du composé (VII), on peut citer des solvants organiques usuels, protiques ou aprotiques, comme les hydrocarbures aromatiques, les hydrocarbures aliphatiques ou cycloaliphatiques, les hydrocarbures halogénés, les alcanols inférieurs comme par exemple le méthanol, l'éthanol, l'isopropanol, l'alcool tertiobutylique, des éthers comme par exemple le diéthyléther, des nitriles comme par exemple l'acétonitrile, des amides comme le diméthylformamide. Avantageusement, la réaction est effectuée à la température de reflux du solvant utilisé. Si nécessaire, la réaction peut être effectuée en vase clos ou sous atmosphère de gaz inerte par exemple d'azote.

La réaction selon l'invention s'effectue dès la mise en présence des trois réactifs de formules (IV), (V) et (VI). Pour cela, il est avantageux de dissoudre l'anilide (IV) et le composé aminoéthylénique (VI) dans un solvant approprié, puis de faire réagir le formaldéhyde (V) sur la solution ainsi obtenue.

Le produit (VII) est séparé du mélange réactionnel par les techniques usuelles, généralement, il cristallise à partir de ce mélange par simple refroidissement. Il peut ensuite être purifié par les méthodes usuelles telles que recristallisation dans un solvant approprié, chromatographie en phase liquide etc. ...

Les produits de départ répondant aux formules (V) et (VI) sont disponibles dans le commerce. L'anilide répondant à la formule (IV) peut être préparée selon la méthode décrite dans «Organic Syntheses, volume 3, page 10», à partir de matières premières appropriées.

Comme indiqué précédemment, le composé (VII) peut si désiré être transformé en le composé (VIII) par déshydrogénation du radical $Q_2$.

Cette déshydrogénation peut être effectuée selon des méthodes en soi connues, décrites notamment dans Chemical Reviews 1972, Vol. 72, No. 1, p. 31, telles que, par exemple:

– par action d'un oxydant tel que l'acide nitreux (généralement formé in situ par action du nitrite de sodium sur l'acide acétique), l'acide chromique, l'acide nitrique, l'iode ou le soufre,

– par chauffage du composé de formule (VII), si nécessaire en présence d'un catalyseur de déshydrogénation tel que par exemple le palladium.

Dans le cas de certains des composés préparés, on a observé de plus que la déshydrogénation du composé (VII) pour donner le composé (VIII) correspondant, s'effectue parfois spontanément dès la température ambiante, sans utilisation de catalyseur, au bout d'un temps plus ou moins long.

Avantageusement, le passage du composé de formule (VII) au composé de formule (VIII) s'effectue par action du nitrite de sodium sur une suspension du composé de formule (VII) dans l'acide acétique. La réaction étant exothermique, il est généralement préféré de refroidir le mélange réactionnel de façon à ce que sa température ne dépasse pas 25 °C.

Le composé de formule (VIII) est séparé du mélange réactionnel par les techniques usuelles. Généralement, le mélange réactionnel est neutralisé par une base minérale telle que l'ammoniaque et le composé par une base minérale telle que l'ammoniaque et le composé (VIII) précipite. Il peut ensuite être purifié par les méthodes usuelles telles que recristallisation dans un solvant approprié (par exemple éthanol), chromatographie en phase liquide etc. ...

Les exemples ci-après, décrits à titre non limitatif, illustrent la préparation des composés selon l'invention ainsi que leurs propriétés herbicides. La structure de ces composés a été confirmée par spectométrie infra-rouge et/ou par spectométrie de résonance magnétique nucléaire (RMN), les spectres ayant été réalisés à 60 mégahertz dans le DMSO, avec l'héxaméthyl disiloxane comme référence interne.

Exemple 1

Préparation de la dihydro-1,4 N-(diéthyl-2,6 phényl) carbamoyl-3-éthoxycarbonyl-5-lutidine-2,6 (composé No 1) de formule:

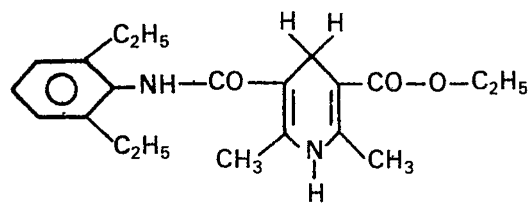

Dans un ballon tricol de 250 ml, muni d'un réfrigérant, d'un thermomètre et d'une agitation mécanique centrale, on charge:

– 23,3 g (0,1 mole) de diéthyl-2,6 acétoacétanilido,

– 12,9 g (0,1 mole) de β-aminocrotonate d'éthyle,

– 50 ml d'éthanol.

Le mélange est agité puis chauffé de la température ambiante jusqu'à 33 °C, jusqu'à dissolution complète des réactifs.

Après refroidissement à 20 °C, on coule dans le mélange, en une minute, 10 ml d'une solution aqueuse à 30 % (en poids) de formaldéhyde (soit environ 0,1 mole). La réaction est exothermique et la température s'élève spontanément de 20 °C jusqu'à 55 °C environ. Le mélange réactionnel est ensuite chauffé à reflux pendant 60 mn.

Après refroidissement jusqu'à température ambiante, filtration, lavage par l'eau du précipité, on obtient 14,5 g d'un mélange comprenant d'une part le produit cherché (composé No 1) et d'autre part, comme sous-produit, la dihydro-1,4-di (éthoxycarbonyl)-3,5 lutidine-2,6, dont la formation résulte de la condensation de deux moles de β-amino-crotonate d'éthyle et d'une mole de formaldéhyde.

Par recristallisation dans 250 ml d'éthanol, on obtient 9 g du composé No 1. Rendement par

rapport à l'acétoacétanilide de départ: 25 %. Point de fusion: 209 °C. Formule brute: $C_{21}H_{28}N_2O_3$.

Le diéthyl-2,6 acétoacétanilide de départ a été préparé par action de la diéthyl-2,6 aniline sur le dicétène, selon la méthode décrite dans «Organic Syntheses, Volume 3, page 10», dans le cas de la préparation de l'acétoacétanilide.

Exemple 2

Préparation des composés No 2 à 22. En opérant selon la méthode décrite à l'exemple précédent, à partir des matières premières appropriées, les composés No 2 à 22 ont été préparés. Les formules et caractéristiques physicochimiques de ces composés sont indiquées dans le tableau A figurant à la fin de la description.

Exemple 3

Préparation du composé No 23. En opérant selon la méthode décrite à l'exemple 1, à partir des matières premières appropriées, le dihydro 1,4 N-(diéthyl-2,6 phényl)-carbamoyl-3 cyano-5 lutidine-2,6 a été préparée:

- point de fusion: 200 °C
- Formule brute: $C_{19}H_{23}N_3O$

Exemple 4

Préparation de la N-(diéthyl-2,6 phényl)-carbamoyl-3-éthoxycarbonyl-5 lutidine-2,6 (composé No 24) ou selon une autre dénomination équivalente: diméthyl-2,6 N-(diéthyl-2,6 phényl)-carbamoyl-3 éthoxycarbonyl-5 pyridine, de formule:

(composé No 24)

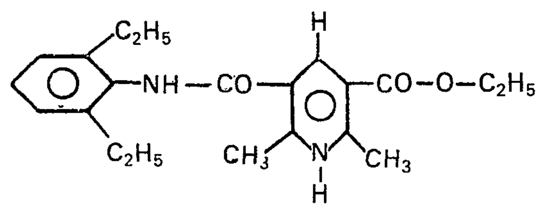

On utilise comme matière de départ la dihydro-1,4 N-(diéthyl-2,6 phényl)-carbamoyl-3 éthoxycarbonyl-5 lutidine-2,6 dont la préparation a été décrite dans l'exemple No 1.

Dans un ballon tricol de 250 ml, muni d'un réfrigérant, d'un thermomètre et d'une agitation mécanique centrale, on charge:

- 5,5 g de la dihydro-1,4 N-(diéthyl-2,6 phényl)-carbamoyl-3 éthoxycarbonyl-5 lutidine-2,6,
- et 55 ml d'acide acétique.

Le mélange réactionnel est refroidi à 16 °C et on ajoute alors par petites portions 1,2 g de nitrite de sodium.

La réaction exothermique est contrôlée par refroidissement à l'aide d'un bain de glace, de manière à ce que la température du mélange réactionnel n'excède pas 25 °C.

Après 30 mn d'agitation vers 20–25 °C, le mélange réactionnel est versé sur de la glace et neutralisé par 75 ml d'ammoniaque concentrée. Le précipité obtenu est filtré et lavé à l'eau. Après filtration sur 120 g de silice, éluée par le mélange chlorure de méthylène/éther 8/2, on obtient 4,2 g du composé cherché (composé No 24).

- point de fusion: 216 °C
- rendement (à partir de la dihydrolutidine): 77%
- formule brute: $C_{21}H_{26}N_2O_3$.

Exemple 5

Préparation des composés No 25 à 53. En opérant selon la méthode décrite à l'exemple précédent, à partir des matières premières appropriées, les composés No 25 à 53 ont été préparés. Les formules et caractéristiques physiochimiques de ces composés sont indiquées dans le tableau A figurant à la fin de la description. Les rendements indiqués dans ce tableau sont calculés par rapport à l'acétoacétanilide de départ.

Exemple 6

Préparation de la N-(diméthyl-2,6 phényl)-carbamoyl-3 cyano-5 lutidine-2,6 (composé No 54).

Ce composé fondant à 243 °C a été préparé en opérant selon la méthode décrite dans l'exemple 4, en utilisant comme matière de départ la dihydro-1,4 N-(diméthyl-2,6 phényl)-carbamoyl-3 cyano-5 lutidine-2,6, elle-même préparée selon la méthode décrite dans l'exemple 3.

Exemple 7

activité herbicide en serre, en prélevée des espèces végétales.

Dans des pots de 9 × 9 × 9 cm remplis de terre agricole légère, on sème un nombre de graines déterminé en fonction de l'espèce végétale et de la grosseur de la graine.

On recouvre ensuite les graines d'une couche de terre d'environ 3 mm d'épaisseur.

Après humidification de la terre, les pots sont traités par pulvérisation d'une quantité de bouillie par pot correspondant à un volume de 500 l/ha et contenant la matière active à la dose considérée.

La bouillie a été préparée en diluant par une quantité d'eau déterminée, de façon à obtenir la concentration voulue, une poudre mouillable ayant la composition pondérale suivante:

| | |
|---|---|
| – matière active à tester | 20% |
| – support inerte solide: kaolinite | 69% |
| – agent tensioactif (défloculant): lignosulfonate de calcium | 5% |
| – agent tensioactif (mouillant): isopropylnaphtalène sulfonate de sodium | 1% |
| – silice antimottante | 5% |

Cette poudre a été obtenue en mélangeant et broyant les ingrédients dans un microniseur, de façon à obtenir une grosseur moyenne de particules inférieure à 40 microns.

Selon la concentration en matière active de la bouillie, la dose de matière active appliquée a été de 2 à 8 kg/ha.

Les pots traités sont ensuite placés dans des bacs destinés à recevoir l'eau d'arrosage, en subirrigation, et maintenus pendant 35 jours à température ambiante sous 70% d'humidité relative.

Au bout de 35 jours, on compte le nombre de plantes vivantes dans les pots traités par la bouillie contenant la matière active à tester et le nombre de plantes vivantes dans un pot témoin traité selon les mêmes conditions, mais au moyen d'une bouillie ne contenant pas de matière active. On détermine ainsi le pourcentage de destruction des plantes traitées par rapport au témoin non traité. Un pourcentage de destruction égal à 100% indique qu'il y a eu destruction complète de l'espèce végétale considérée et un pourcentage de 0% indique que le nombre de plantes vivantes dans le pot traité est identique à celui dans le pot témoin.

Pour cet essai, les espèces végétales utilisées ont été les suivantes:

| Adventices | Symbole utilisé |
|---|---|
| Folle avoine (Avena fatua) | FAV |
| Digitaire (Digitaria sanguinalis) | DIG |
| Panisse (Echinochloa crus-galli) | PAN |
| Ray-grass (Lolium multiflorum) | RAY |
| Sétaire (Setaria faberii) | SET |
| Vulpin (Alopecurus myosuroides) | VUL |
| Chénopode (Chenopodium sp) | CHE |
| Morelle (Solanum nigrum) | MOR |
| Moutarde (Sinapis arvensis) | MOU |
| Stellaire (Stellaria media) | STE |
| **Cultures** | |
| Coton (Gossypium barbadense) | COT |
| Tournesol (Helianthus annuus) | TOU |

Les résultats observés sont indiqués dans le tableau B figurant à la fin de la description.

Ces résultats montrent l'excellente activité herbicide des composés selon l'invention sur la plupart des adventices traitées, tant graminées que dicotylédones ainsi que leur sélectivité vis-à-vis des cultures considérées.

Pour leur emploi dans la pratique, les composés selon l'invention sont rarement utilisés seuls, mais le plus souvent sous forme de compositions qui font également partie de l'invention et qui contiennent, en général, en plus de la matière active selon l'invention, un ou plusieurs supports, solides ou liquides, acceptables en agriculture et/ou un ou plusieurs agents tensioactifs, également acceptables en agriculture.

Par le terme «support», dans le présent exposé, on désigne une matière organique ou minérale, naturelle ou synthétique, avec laquelle la matière active est associée pour faciliter son application sur les plantes ou sur le sol. Le support peut être solide (argiles, silicates naturels ou synthétiques, silice, résines, cires, engrais solides etc...) ou liquide (eau, alcools, fraction de pétrole, hydrocarbures aromatiques ou paraffiniques, hydrocarbures chlorés, gaz liquéfiés etc...).

L'agent tensioactif peut être un agent émulsionant, dispersant, défloculant ou mouillant, de type ionique ou non ionique. On peut citer par exemple des sels d'acides polyacryliques, des sels d'acides lignosulfoniques, des sels d'acides phénolsulfoniques ou naphtalènesulfoniques, des polycondensats d'oxyde d'éthylène sur des alcools gras ou sur les acides gras ou sur des amines grasses, des phénols substitués (notamment des alkylphénols ou des arylphénols), des sels d'esters d'acides sulfosucciniques, des dérivés de la taurine (notamment des alkyltaurates), des esters phosphoriques d'alcools ou de phénols polyoxyéthylés.

D'une façon générale, les compositions selon l'invention contiennent habituellement de 0,001 à 95% environ (en poids) d'un ou plusieurs composés selon l'invention. Leur teneur en agent tensioactif est généralement comprise entre 0 et 20% en poids.

Toutefois, ces compositions peuvent contenir, de plus, toutes sortes d'autres ingrédients tels que, par exemple, des épaississants, de agents thixotropes, des colloides protecteurs, des adhésifs, des agents de pénétration, des stabilisants, etc... ainsi que d'autres matières actives connues à propriétés pesticides (notamment herbicides, fongicides et insecticides) ou à propriétés favorisant la croissance des plantes (notamment des engrais) ou à propriétés régulatrices de la croissance des plantes. Plus généralement, les composés selon l'invention peuvent être associés à tous les additifs solides ou liquides correspondant aux techniques usuelles de préparation des compositions pesticides.

Les compositions selon l'invention peuvent être préparées sous la forme de poudres mouillables, de poudres pour poudrage, granulés, solutions, de concentrés émulsionnables, d'émulsions, de concentrés en suspension et d'aérosols.

Les poudres mouillables, ou poudres à pulvériser contiennent habituellement de 20 à 95% en poids de matière active et contiennent généralement, en plus d'un support solide, de 0 à 5% en poids d'agent mouillant, de 3 à 10% en poids d'un ou de stabilisants et/ou d'autres additifs, comme des agents de pénétration, des adhésifs ou des agents antimottants, colorants etc...

Elles sont préparées en mélangeant les constituants dans des mélangeurs et en les broyant dans des moulins ou autres broxeurs appropriés, broyeurs à air par exemple, de façon à obtenir la granulométrie voulue.

A titre d'exemple, voici la composition d'une poudre mouillable à 80% (pourcentages en poids):

| | |
|---|---|
| – matière active (composé No ) | 80% |
| – alkylnaphtalène-sulfonate de sodium | 2% |
| – lignosulfonate de sodium | 2% |
| – silice antimottante | 3% |
| – kaolinite | 13% |

Un autre exemple de poudre mouillable est donné ci-après:

| | |
|---|---|
| – matière active (composé No 3) | 50% |
| – alkylnaphtalène sulfonate de sodium | 2% |
| – méthyl cellulose de faible viscosité | 2% |
| – terre de diatomées | 46% |

Un autre exemple de poudre mouillable est donné ci-après:

| | |
|---|---|
| – matière active (composé No 49) | 90% |
| – dioctylsulfosuccinate de sodium | 0,2% |
| – silice synthétique | 9,8% |

Les granulés destinés à être disposés sur le sol sont habituellement préparés de manière qu'ils aient des dimensions comprises entre 0,1 et 2 mm et ils peuvent être fabriqués par agglomération ou imprégnation. En général, les granulés contiendront de 0,5 à 25% de matière active et de 0 à 10% en poids d'additifs comme des stabilisants, des agents de modification à libération lente, des liants et des solvants.

Les concentrés émulsionnables applicables en pulvérisation contiennent habituellement de 10 à 50% en poids/volume de matière active. En plus de la matière active et du solvant, ils peuvent également contenir, si nécessaire, de 2 à 20% en poids/volume d'additifs appropriés tels que des agents tensioactifs, des stabilisants, des agents de pénétration, des inhibiteurs de corrosion, des colorants, des adhésifs.

Les concentrés en suspension, également applicables en pulvérisation, sont préparés de manière que l'on obtienne un produit fluide stable ne se déposant pas et ils contiennent habituellement de 10 à 75% en poids de matière active, de 0,5 à 15% en poids d'agents tensioactifs, de 0,1 à 10% en poids d'agents thixotropes, de 0 à 10% d'additifs appropriés, comme des antimousses, des inhibiteurs de corrision, des stabilisants, des agents de pénétration et des adhésifs et comme support, de l'eau ou un liquide organique dans lequel la matière active est sensiblement insoluble: certaines matières solides organiques ou des sels minéraux peuvent être dissouts dans le support pour aider à empêcher la sédimentation ou comme antigels pour l'eau.

Les dispersions et émulsions aqueuses, obtenues en diluant par de l'eau des compositions mentionnées plus haut, notamment les poudres mouillables et concentrés émulsionnables selon l'invention, sont également comprises dans le cadre général de la présente invention. Les émulsions ainsi obtenues peuvent être du type eau-dans-l'huile ou du type huile-dans-l'eau et elles peuvent avoir une consistance épaisse comme celle d'une mayonnaise.

Toutes ces dispersions et émulsions aqueuses ou bouillies sont applicables aux cultures à désherber par tout moyen convenable, principalement par pulvérisation, à des doses qui sont généralement de l'ordre de 500 à 1 000 litres de bouillies à l'hectare.

Comme indiqué plus haut, l'invention concerne également un procédé de désherbage de cultures telles que le coton et le tournesol, selon lequel on applique sur les plantes et/ou sur le sol de la zone à désherber une quantité efficace d'au moins un des composés selon l'invention. Généralement, des quantités de matière active allant de 0,5 à 10 kg/ha donnent de bons résultats, étant entendu que le choix de la quantité de matière active à utiliser est fonction de l'intensité du problème à résoudre, des conditions climatiques et de la culture considérée. Le traitement est en général effectué en prélevée des cultures et adventices ou en présemis des cultures avec incorporation dans le sol, bien que dans certains cas, selon le composé utilisé de bons résultats puissent également être obtenus par des traitements de post-levée.

**Tableau A**

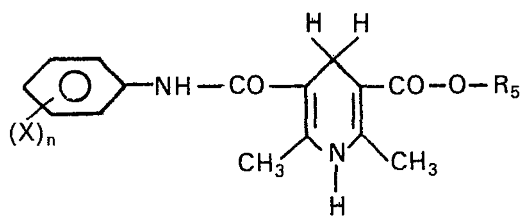

| Composé No | 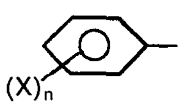 | $R_5$ | Formule brute | Point de Fusion (°C) | Rendement % |
|---|---|---|---|---|---|
| 2 | 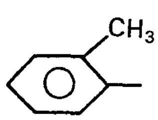 | $C_2H_5$ | $C_{18}H_{22}N_2O_3$ | 164 | 29 |
| 3 | 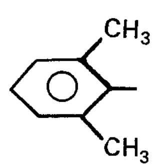 | $C_2H_5$ | $C_{19}H_{24}N_2O_3$ | 159 | 20 |

**Tableau A** (suite)

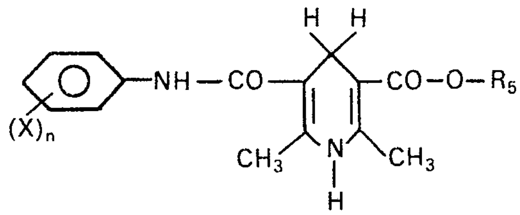

| Composé No | 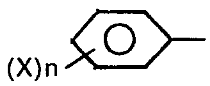 | $R_5$ | Formule brute | Point de Fusion (°C) | Rendement % |
|---|---|---|---|---|---|
| 4 | 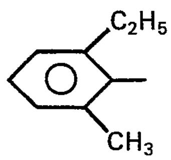 | $C_2H_5$ | $C_{20}H_{26}N_2O_3$ | 163 | 32 |
| 5 | 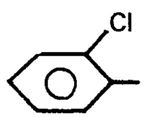 | $C_2H_5$ | $C_{17}H_{19}ClN_2O_3$ | 100 | 30 |
| 6 | 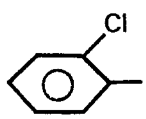 | $CH_3$ | $C_{16}H_{17}ClN_2O_3$ | 125 | 67 |
| 7 | 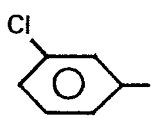 | $C_2H_5$ | $C_{17}H_{19}ClN_2O_3$ | 190 | 26 |
| 8 | 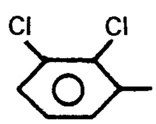 | $C_2H_5$ | $C_{17}H_{18}Cl_2N_2O_3$ | 136 | 30 |
| 9 | 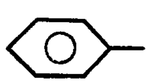 | $-C_2H_5$ | $C_{17}H_{20}N_2O_3$ | 142 | 19 |
| 10 | 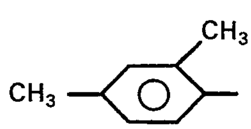 | $-C_2H_5$ | $C_{19}H_{24}N_2O_3$ | 175 | 21 |
| 11 | 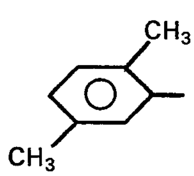 | $-C_2H_5$ | $C_{19}H_{24}N_2O_3$ | 165 | 17 |
| 12 | 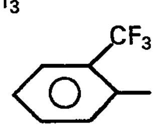 | $-C_2H_5$ | $C_{18}H_{19}F_3N_2O_3$ | 145 | 25 |

**Tableau A** (suite)

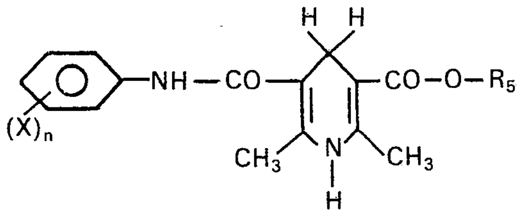

| Composé No | 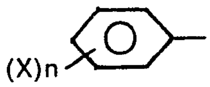 | $R_5$ | Formule brute | Point de Fusion (°C) | Rendement % |
|---|---|---|---|---|---|
| 13 | 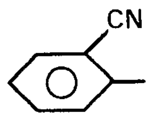 | $-C_2H_5$ | $C_{18}H_{19}N_3O_3$ | 136 | 47 |
| 14 | 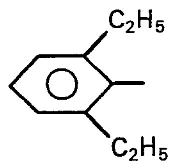 | $-CH_3$ | $C_{20}H_{26}N_2O_3$ | 211 | 18 |
| 15 | 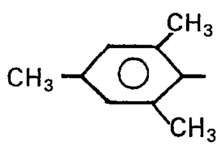 | $-C_2H_5$ | $C_{20}H_{26}N_2O_3$ | 165 | 33 |
| 16 | 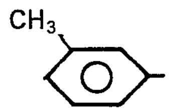 | $-C_2H_5$ | $C_{18}H_{22}N_2O_3$ | 140 | 26 |
| 17 | 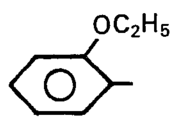 | $-C_2H_5$ | $C_{19}H_{24}N_2O_4$ | 117 | 31 |
| 18 | 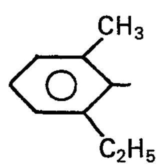 | $-CH_3$ | $C_{19}H_{24}N_2O_3$ | 184 | 38 |
| 19 | 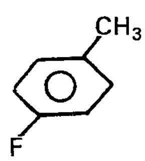 | $-C_2H_5$ | $C_{18}H_{21}FN_2O_3$ | 148 | 27 |
| 20 | 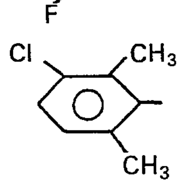 | $-CH_3$ | $C_{18}H_{21}ClN_2O_3$ | 175 | 20 |
| 21 | 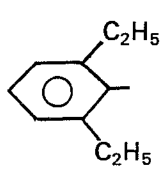 | $-(CH_2)_2CH_3$ | $C_{22}H_{30}N_2O_3$ | 180–185 | 89 |
| 22 | 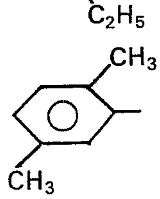 | $-C_2H_5$ | $C_{19}H_{24}N_2O_3$ | 166 | 17 |

**Tableau A** (suite)

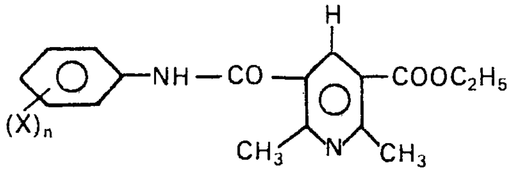

| Composé No | 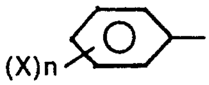 | Formule brute | Point de Fusion (°C) | Rendement % |
|---|---|---|---|---|
| 25 | 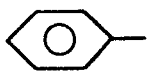 | $C_{17}H_{18}N_2O_3$ | 105 | 11 |
| 26 | 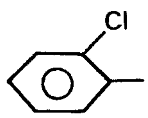 | $C_{17}H_{17}ClN_2O_3$ | 136 | 40 |
| 27 | 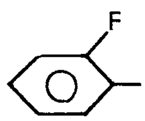 | $C_{17}H_{17}FN_2O_3$ | 137 | – |
| 28 | 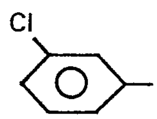 | $C_{17}H_{17}ClN_2O_3$ | 88 | 23 |
| 29 | 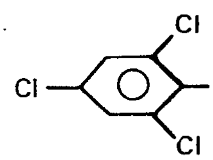 | $C_{17}H_{15}Cl_3N_2O_3$ | 189 | 51 |
| 30 | 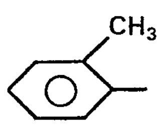 | $C_{18}H_{20}N_2O_3$ | 132 | 12 |
| 31 | 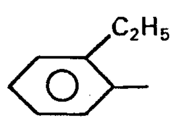 | $C_{19}H_{22}N_2O_3$ | 188 | 4 |
| 32 | 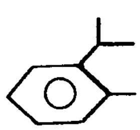 | $C_{20}H_{24}N_2O_3$ | 133 | – |
| 33 | 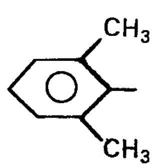 | $C_{19}H_{22}N_2O_3$ | 187 | 17 |
| 34 | 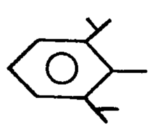 | $C_{23}H_{30}N_2O_3$ | 159 | – |

**Tableau A** (suite)

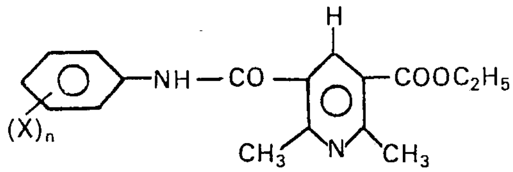

| Composé No | 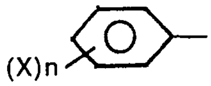 | Formule brute | Point de Fusion (°C) | Rendement % |
|---|---|---|---|---|
| 35 | 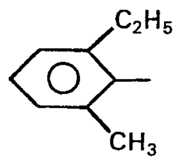 | $C_{20}H_{24}N_2O_3$ | 203 | 19 |
| 36 | 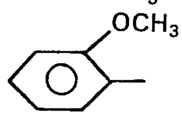 | $C_{18}H_{20}N_2O_4$ | 86 | – |
| 37 | 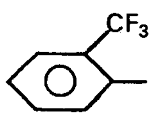 | $C_{18}H_{17}F_3N_2O_3$ | 175 | 18,5 |
| 38 | 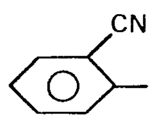 | $C_{18}H_{16}Cl_2N_2O_3$ | 174 | 23,5 |
| 39 | 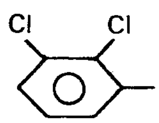 | $C_{17}H_{16}Cl_2N_2O_3$ | 162 | 15 |

**Tableau A** (suite)

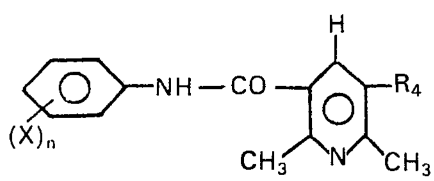

| Composé No | 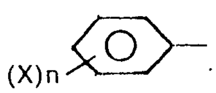 | $R_4$ | Formule brute | Point de Fusion (°C) | Rendement % |
|---|---|---|---|---|---|
| 40 | 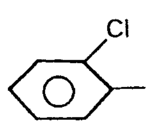 | $-COO-CH_3$ | $C_{16}H_{15}ClN_2O_3$ | 142 | 49 |
| 41 | 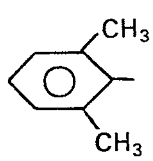 | $-CN$ | $C_{17}H_{17}N_3O$ | 243 | 65 |
| 42 | 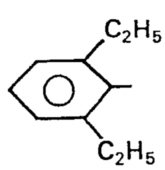 | $-COOCH_3$ | $C_{20}H_{24}N_2O_3$ | 192 | 25 |

Tableau A (suite)

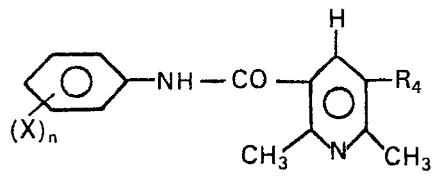

| Composé No | 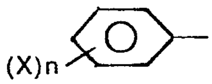 | $R_4$ | Formule brute | Point de Fusion (°C) | Rendement % |
|---|---|---|---|---|---|
| 43 | 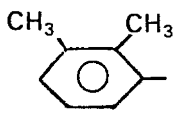 | $-COOC_2H_5$ | $C_{19}H_{22}N_2O_3$ | 185 | 12 |
| 44 | 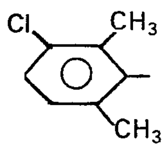 | $-COOC_2H_5$ | $C_{19}H_{21}ClN_2O_3$ | 205 | 15 |
| 45 | 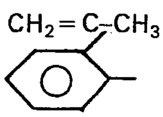 | $-COOC_2H_5$ | $C_{20}H_{22}N_2O_3$ | 116 | 10 |
| 46 | 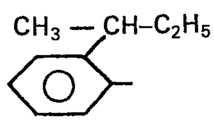 | $-COOC_2H_5$ | $C_{21}H_{26}N_2O_3$ | 95–96 | 20 |
| 47 | 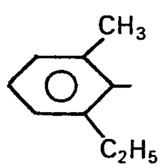 | $-COOCH_3$ | $C_{19}H_{22}N_2O_3$ | 188 | 34 |
| 48 | 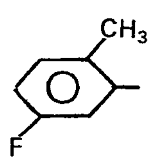 | $-COOC_2H_5$ | $C_{18}H_{19}FN_2O_3$ | 156 | 25 |
| 49 | 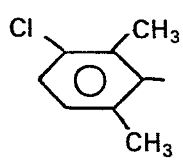 | $-COOCH_3$ | $C_{18}H_{19}ClN_2O_3$ | 207 | 30 |
| 50 | 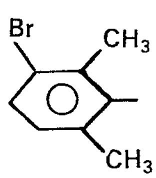 | $-COOC_2H_5$ | $C_{19}H_{21}BrN_2O_3$ | 208 | 14 |
| 51 | 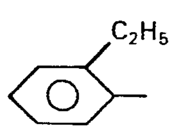 | $-COOC_2H_5$ | $C_{19}H_{22}N_2O_3$ | 188 | 20 |

Tableau A (suite)

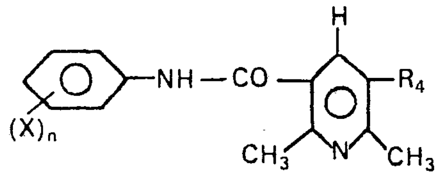

| Composé No | 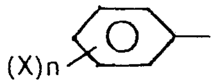 | $R_4$ | Formule brute | Point de Fusion (°C) | Rendement % |
|---|---|---|---|---|---|
| 52 | 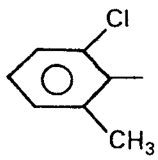 | $-COOC_2H_5$ | $C_{18}H_{19}ClN_2O_3$ | 178 | 21 |
| 53 | 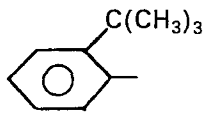 | $-COOC_2H_5$ | $C_{21}H_{26}N_2O_3$ | 70 | 50 |

**Tableau B**

Activité herbicide en pré-levée – 100 = destruction complète
0 = aucune action herbicide

| Composé No | Dose kg/ha | ADVENTICES FAV | DIG | PAN | RAY | SET | VUL | CHE | MOR | MOU | STE | CULTURES COT | TOU |
|---|---|---|---|---|---|---|---|---|---|---|---|---|---|
| | 2 | 30 | 100 | 80 | 80 | 100 | 100 | 100 | 100 | 15 | 100 | 0 | 0 |
| 1 | 4 | 90 | 100 | 100 | 80 | 100 | 100 | 100 | 100 | 50 | 100 | 0 | 0 |
| | 8 | 90 | 100 | 100 | 100 | 100 | 100 | 100 | 100 | 95 | 100 | – | 0 |
| 2 | 4 | 20 | 100 | 80 | 30 | 100 | 100 | 90 | 100 | 50 | 60 | 0 | 15 |
| 3 | 4 | 50 | 100 | 80 | 20 | 100 | 30 | 100 | 100 | 100 | 100 | 0 | 0 |
| 4 | 4 | 60 | 100 | 100 | 100 | 80 | 90 | 100 | 100 | 95 | 100 | 0 | 0 |
| | 8 | 60 | 100 | 100 | 100 | 90 | 100 | 100 | 100 | 100 | 100 | 0 | 20 |
| 5 | 8 | 40 | – | 40 | 30 | – | – | 100 | – | 30 | – | – | – |
| 6 | 8 | 90 | 100 | 0 | 80 | 100 | 100 | 100 | 100 | 30 | 100 | 0 | 0 |
| 7 | 8 | 70 | – | 30 | 10 | – | – | 100 | – | 30 | – | – | – |
| 8 | 4 | 20 | 90 | 30 | 40 | 100 | 100 | 100 | 100 | 20 | 100 | 0 | 0 |
| 9 | 8 | 60 | – | 30 | 20 | – | – | 100 | – | 30 | – | – | – |
| 10 | 8 | 10 | – | 75 | 0 | – | – | 80 | – | 20 | – | – | – |
| 11 | 8 | 40 | – | 100 | 90 | – | – | 100 | – | 100 | – | – | – |
| 12 | 8 | 20 | – | 30 | 30 | – | – | 100 | – | 50 | – | – | – |
| 13 | 8 | 10 | – | 75 | 20 | – | – | 90 | – | 0 | – | – | – |
| 14 | 2 | 5 | 100 | 80 | 90 | 100 | 100 | 100 | 100 | 100 | 100 | 10 | 0 |
| 15 | 8 | 80 | – | 100 | 90 | – | – | 100 | – | 100 | – | – | – |

**Tableau B** (suite)

Activité herbicide en pré-levée – 100 = destruction complète
0 = aucune action herbicide

| Composé | Dose | ADVENTICES | | | | | | | | | | CULTURES | |
|---|---|---|---|---|---|---|---|---|---|---|---|---|---|
| No | kg/ha | FAV | DIG | PAN | RAY | SET | VUL | CHE | MOR | MOU | STE | COT | TOU |
| 16 | 8 | 50 | – | 80 | 60 | – | – | 100 | – | 100 | – | – | – |
| 17 | 8 | 80 | – | 95 | 95 | – | – | 100 | – | 20 | – | – | – |
| 18 | 8 | 100 | – | 100 | 100 | – | – | 100 | – | 100 | – | – | – |
| 19 | 8 | 40 | – | 95 | 90 | – | – | 100 | – | 100 | – | – | – |
| 20 | 8 | 100 | – | 100 | 100 | – | – | 100 | – | 100 | – | – | – |
| 22 | 8 | 30 | 95 | 100 | 90 | 100 | 95 | 100 | 100 | 60 | 100 | – | – |
| 23 | 8 | 90 | – | 100 | 95 | – | – | 100 | – | 100 | – | – | – |
| 24 | 4 | 0 | 100 | 10 | 100 | 0 | 50 | 100 | 100 | 0 | 100 | 0 | 0 |
| 25 | 8 | 10 | 90 | 50 | 5 | 100 | 20 | 100 | 100 | 30 | 0 | 0 | 0 |
| 26 | 8 | 50 | 100 | 50 | 20 | 100 | 80 | 100 | 100 | 30 | 15 | – | – |
| 27 | 4 | 5 | 80 | 30 | 10 | 100 | 20 | 80 | 100 | 10 | 5 | 0 | 0 |
| | 8 | 20 | 100 | 50 | 20 | 100 | 40 | 100 | 100 | 50 | 5 | 0 | – |
| 28 | 8 | 0 | 50 | 20 | 5 | 100 | 15 | 100 | 100 | 5 | 80 | 0 | – |
| 29 | 8 | 0 | – | 0 | 0 | – | – | 100 | – | 20 | – | – | – |
| 30 | 4 | 5 | 100 | 20 | 50 | 100 | 80 | 70 | 100 | 30 | 20 | – | 0 |
| | 8 | 50 | 100 | 70 | 60 | 100 | 100 | 95 | 100 | 40 | 40 | – | – |
| 31 | 8 | 50 | – | 90 | 70 | – | – | 100 | – | 80 | – | – | – |
| 32 | 8 | 10 | – | 75 | 30 | – | – | 100 | – | 60 | – | – | – |
| | 2 | 5 | 100 | 50 | 10 | 100 | 20 | 100 | 100 | 25 | 95 | 0 | 0 |
| 33 | 4 | 50 | 100 | 50 | 50 | 100 | 20 | 100 | 100 | 95 | 100 | 0 | – |
| | 8 | 80 | 100 | 80 | 50 | 100 | 70 | 100 | 100 | 95 | 100 | 0 | |
| 34 | 8 | 0 | – | 0 | 0 | – | – | 80 | – | 30 | – | – | – |
| 35 | 4 | 0 | 100 | 40 | 30 | 30 | 100 | 60 | 100 | 60 | 100 | – | – |
| 36 | 8 | 0 | – | 20 | 20 | – | – | 60 | – | 50 | – | – | – |
| 37 | 8 | 0 | 20 | 40 | 0 | 60 | 0 | 90 | 100 | 0 | 50 | 0 | 0 |
| 38 | 8 | 50 | – | 80 | 50 | – | – | 90 | 20 | – | – | – | – |
| 39 | 8 | 0 | 20 | 20 | 0 | 95 | 20 | 90 | 100 | 0 | 100 | 0 | 0 |
| 40 | 8 | 90 | – | 30 | 80 | – | – | 30 | – | 60 | – | – | – |
| 41 | 8 | 90 | – | 30 | 30 | – | – | 100 | – | 100 | – | – | – |

**Tableau B** (suite)

Activité herbicide en pré-levée – 100 = destruction complète
0 = aucune action herbicide

| Composé | Dose | ADVENTICES | | | | | | | | | | CULTURES | |
|---|---|---|---|---|---|---|---|---|---|---|---|---|---|
| No | kg/ha | FAV | DIG | PAN | RAY | SET | VUL | CHE | MOR | MOU | STE | COT | TOU |
| 42 | 1 | 0 | 100 | 60 | 80 | 100 | 90 | 100 | 100 | 60 | 100 | 0 | 0 |
| | 4 | 60 | 100 | 95 | 95 | 100 | 100 | 100 | 100 | 80 | 100 | 0 | 0 |
| 43 | 8 | 90 | – | 100 | 60 | – | – | 100 | – | 100 | – | – | – |
| 44 | 8 | 95 | – | 100 | 100 | – | – | 100 | – | 100 | – | – | – |
| 45 | 8 | 20 | – | 20 | 20 | – | – | 100 | – | 20 | – | – | – |
| 46 | 8 | 20 | – | 30 | 20 | – | – | 100 | – | 100 | – | – | – |
| 47 | 8 | 100 | – | 100 | 100 | – | – | 100 | – | 100 | – | – | – |
| 48 | 8 | 90 | – | 100 | 100 | – | – | 100 | – | 100 | – | – | – |
| 49 | 8 | 98 | – | 100 | 100 | – | – | 100 | – | 100 | – | – | – |
| 50 | 8 | 30 | – | 98 | 80 | – | – | 100 | – | 100 | – | – | – |
| 51 | 8 | 5 | 100 | 60 | 20 | 100 | 60 | 100 | 100 | 20 | 100 | 0 | 0 |
| 52 | 8 | 0 | 98 | 90 | 10 | 100 | 30 | 100 | 100 | 80 | 95 | 0 | 0 |
| 53 | 8 | 0 | 95 | 0 | 0 | 95 | 20 | 100 | 100 | 0 | 100 | 0 | 0 |
| 54 | 8 | 5 | 98 | 20 | 10 | 100 | 5 | 100 | 100 | 20 | 0 | 0 | 0 |

**Revendications pour les Etats contractants: BE, CH, DE, FR, GB, IT, LI, LU, NL, SE**

1. Dérivé de l'aniline caractérisé en ce qu'il répond à la formule générale:

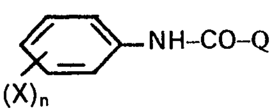

(I)

dans laquelle:

– X représente un atom ou radical choisi parmi:

– les atomes d'halogènes,

– les radicaux alkyle contenant de 1 à 6 atomes de carbone,

– les radicaux alkoxy contenant de 1 à 6 atomes de carbone,

– les radicaux alcényle contenant de 2 à 6 atomes de carbone,

– les radicaux alcényloxy contenant de 2 à 6 atomes de carbone,

– les radicaux halogénoalkyle contenant de 1 à 6 atomes de carbone, et substitués par un ou plusieurs halogènes,

– le radical amino, éventuellement substitué par un ou deux radicaux alkyle, identiques ou différents, contenant chacun de 1 à 6 atomes de carbone, ou par le radical $-CO-R_8$ dans lequel $R_8$ représente un radical alkyle contenant de 1 à 6 atomes de carbone, alkoxy contenant de 1 à 6 atomes de carbone, alkylamino contenant de 1 à 6 atomes de carbone ou dialkylamino dans lequel chacune des parties alkyle, identiques ou différentes, comporte de 1 à 6 atomes de carbone,

– le radical nitro,

– et le radical cyano.

– n est un nombre entier de 0 à 5, étant entendu que lorsque n est supérieur à 1, les substituants X peuvent être identiques ou différents.

– Q représente un radical répondant à l'une des formules ci-après:

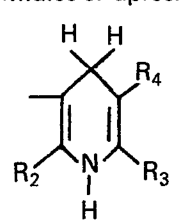

(IA)

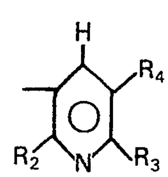

(IB)

dans laquelle:

– $R_2$ et $R_3$, identiques ou différents, représentent chacun un atome d'hydrogène ou un radical alkyle contenant de 1 à 6 atomes de carbone,

– $R_4$ représente le radical cyano ou un radical $-COR_5$ dans lequel $R_5$ représente un hydroxy ou un radical $-OR_6$ dans lequel $R_6$ représente un radical alkyle contenant de 1 à 6 atomes de carbone,

– et, les sels acceptables en agriculture de ce dérivé,

– ainsi que, lorsque Q représente le radical IA, les formes tautomères de ce dérivé.

2. Composé selon la revendication 1, caractérisé en ce que:

– $R_2$ et $R_3$, identiques ou différents, représentent l'atome d'hydrogène ou un radical alkyle comportant de 1 à 3 atomes de carbone,

– $R_4$ représente un radical alkoxycarbonyle comportant de 2 à 6 atomes de carbone ou le radical cyano,

– X représente un atome ou radical choisi parmi les atomes d'halogènes, les radicaux alkyle comportant de 1 à 4 atomes de carbone, alkoxy comportant de 1 à 4 atomes de carbone, halogénoalkyle comportant de 1 à 4 atomes de carbone et cyano,

– n est un nombre entier pouvant être égal à 0, 1, 2, 3 ou 4, étant entendu que lorsque n est supérieur à 1, les substituants X peuvent être soit identiques, soit différents.

3. Composé selon la revendication 2, caractérisé en ce qu'il répond à la formule:

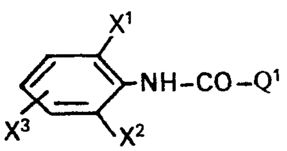

(III)

dans laquelle:

– $X_1$ représente un radical alkyle comportant de 1 à 3 atomes de carbone,

– $X_2$ représente l'atome d'hydrogène ou un radical alkyle comportant de 1 à 3 atomes de carbone,

– $X_3$ représente un atome d'hydrogène ou d'halogṅe ou un radical alkyle comportant de 1 à 3 atomes de carbone,

– $Q_1$ représente un radical répondant à l'une des formules ci-après:

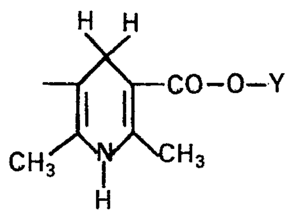

(IIIA)

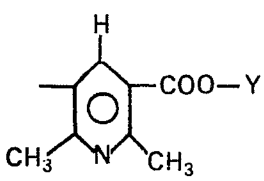

(IIIB)

dans laquelle:

– Y représente un radical alkyl comportant de 1 à 4 atomes de carbone.

4. Composition herbicide, caractérisée en ce qu'elle contient comme matière active au moins un composé selon l'une des revendications 1 à 3.

5. Composition selon la revendication 4, caractérisée en ce qu'elle contient, en poids, de 0,001% à 95% de matière active.

6. Procédé de préparation d'un composé selon la revendication 1, caractérisé en ce que l'on fait réagir ensemble:

a – l'anilide de formule:

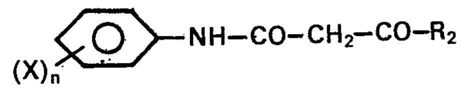

dans laquelle X, n et $R_2$ ont la même signification que dans la revendication 1,

b – le formaldéhyde,

c – et le dérivé aminoéthylénique de formule:

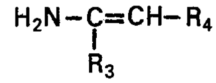

dans laquelle $R_3$ et $R_4$ ont la même signification que dans la revendication 1 pour donner le composé de formule (VII):

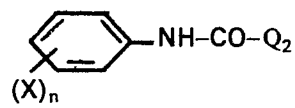

(VII)

dans laquelle X et n ont la même signification que précédemment et $Q_2$ représente le radical:

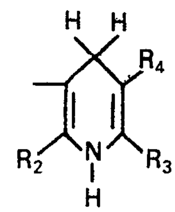

dans lequel $R_2$, $R_3$, $R_4$ ont même signification que précédemment, et en ce que, éventuellement, on transforme, en une deuxième étape, par déshydrogénation du radical $Q_2$, le composé de formule (VII) en un composé de formule (VIII):

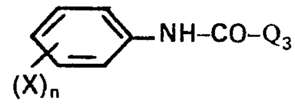

(VIII)

dans laquelle X et n ont la même signification que précédemment et $Q_3$ représente le radical:

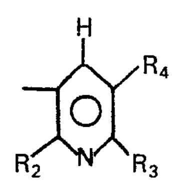

dans lequel $R_2$, $R_3$ et $R_4$ ont même signification que précédemment.

7. Procédé selon la revendication 6, caractérisé en ce que la réaction selon la première étape du procédé est effectuée en milieu solvant organique, à une température comprise entre 15 °C et 100 °C.

8. Procédé selon la revendication 6, caractérisé en ce que la deshydrogénation selon la deuxième étape du procédé est effectuée par action du nitrite de sodium sur une solution dans l'acide acétique du composé résultant de la première étape dudit procédé.

9. Procédé de désherbage de cultures de coton et tournesol, caractérisé en ce qu'on applique sur les zones cultivées, en prélevée des cultures ou en présemis des cultures avec incorporation dans le sol, une quantité efficace d'au moins un composé selon l'une des revendications 1 à 3.

**Revendications pour l'Etat contractant: AT**

1. Composition herbicide, caractérisée en ce qu'elle contient comme matière active au moins un composé répondant à la formule I:

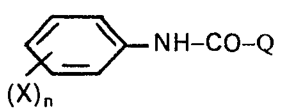

(I)

dans laquelle:

– X représente un atome ou radical choisi parmi:

– les atomes d'halogènes,

– les radicaux alkyle contenant de 1 à 6 atomes de carbone,

– les radicaux alkoxy contenant de 1 à 6 atomes de carbone,

– les radicaux alcényle contenant de 2 à 6 atomes de carbone,

– les radicaux alcényloxy contenant de 2 à 6 atomes de carbone,

– les radicaux halogénoalkyle contenant de 1 à 6 atomes de carbone, et substitués par un ou plusieurs halogènes,

– le radical amino, éventuellement substitué par un ou deux radicaux alkyle, identiques ou différents, contenant chacun de 1 à 6 atomes de carbone, ou par le radical $-CO-R_8$ dans lequel $R_8$ représente un radical alkyle contenant de 1 à 6 atomes de carbone, alkoxy contenant de 1 à 6 atomes de carbone ou dialkylamino dans lequel chacune des parties alkyle, identiques ou différentes, comporte de 1 à 6 atomes de carbone, le radical nitro,

– et le radical cyano,

– n est un nombre entier de 0 à 5, étant entendu que lorsque n est supérieur à 1, les substituants X peuvent être identiques ou différents.

– Q représente un radical répondant à l'une des formules IA et IB:

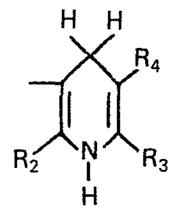

(IA)

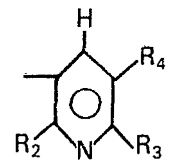

(IB)

dans lesquelles:

– $R_2$ et $R_3$, identiques ou différents, représentent chacun un atome d'hydrogène ou un radical alkyle contenant de 1 à 6 atomes de carbone,

– $R_4$ représente le radical cyano ou un radical $-COR_5$ dans lequel $R_5$ représente un hydroxy ou un radical $-OR_6$ dans lequel $R_6$ représente un radical alkyle contenant de 1 à 6 atomes de carbone,

– ou un sel acceptable en agriculture de ce dérivé,

– ou, lorsque Q représente le radical IA, une forme tautomère de ce dérivé.

2. Composition selon la revendication 1, caractérisée en ce qu'elle contient comme matière active au moins un composé selon la formule I de la revendication 1, dans laquelle:

– X représente un atome ou radical choisi parmi les atomes d'halogènes, les radicaux alkyle comportant de 1 à 4 atomes de carbone, alkoxy comportant de 1 à 4 atomes de carbone, halogénoalkyle comportant de 1 à 4 atomes de carbone et cyano,

– n est un nombre entier pouvant être égal à 0, 1, 2, 3 ou 4, étant entendu que lorsque n est supérieur à 1, les substituants X peuvent être soit identiques, soit différents,

– Q représente un radical répondant à l'une des formules IA et IB de la revendication 1 dans laquelle:

– $R_2$ et $R_3$, identiques ou différents, représentent l'atome d'hydrogène ou un radical alkyle comportant de 1 à 3 atomes de carbone,

– $R_4$ représente un radical alkoxycarbonyle comportant de 2 à 6 atomes de carbone ou le radical cyano.

3. Composition selon la revendication 2, caractérisée en ce qu'elle contient comme matière active au moins cun composé répondant à la formule III

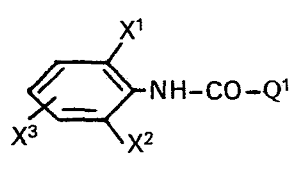

(III)

dans laquelle:

– $X_1$ représente un radical alkyle comportant de 1 à 3 atomes de carbone,

– $X_2$ représente l'atome d'hydrogène ou un radical alkyle comportant de 1 à 3 atomes de carbone,

– $X_3$ représente un atome d'hydrogène ou d'halogène ou un radical alkyle comportant de 1 à 3 atomes de carbone,

– $Q_1$ représente un radical répondant à l'une des formules IIIA et IIIB ci-après:

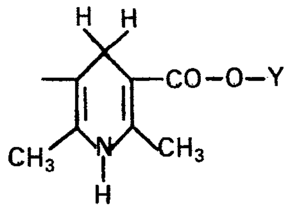
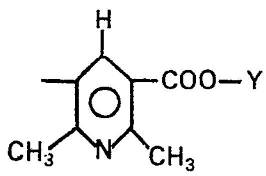

(IIIA) (IIIB)

dans lesquelles:

– Y représente un radical alkyle comportant de 1 à 4 atomes de carbone.

4. Composition selon l'une des revendications 1 à 3 caractérisée en ce qu'elle contient, en poids, de 0,001% à 95% de matière active.

5. Procédé de préparation d'un composé selon la formule I de la revendication 1, caractérisé en ce que l'on fait réagir ensemble:

a – l'anilide de formule:

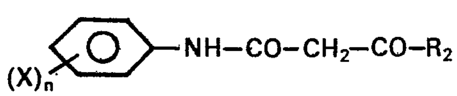

dans laquelle X, n et $R_2$ ont la même signification que dans la revendication 1,

b – la formaldéhyde

c – et le dérivé aminoéthylénique de formule:

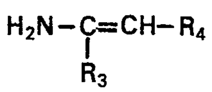

dans laquelle $R_3$ et $R_4$ ont la même signification que dans la revendication 1 pour donner le composé de formule (VII):

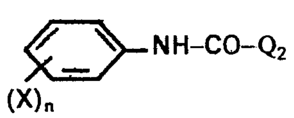

(VII)

dans laquelle X et n ont la même signification que précédemment et $Q_2$ représente le radical:

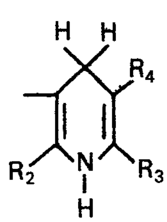

dans lequel $R_2$, $R_3$, $R_4$ ont même signification que précédemment, et en ce que, éventuellement, on transforme, en une deuxième étape, par déshydrogénation du radical $Q_2$, le composé de formule (VII) en un composé de formule (VIII):

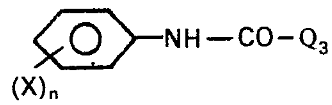

(VIII)

dans laquelle X et n ont la même signification que précédemment et $Q_3$ représente le radical:

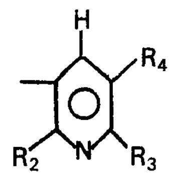

dans lequel $R_2$, $R_3$ et $R_4$ ont même signification que précédemment.

6. Procédé selon la revendication 5, caractérisé en ce que la réaction selon la première étape du procédé est effectuée en milieu solvant organique, à une température comprise entre 15 °C et 100 °C.

7. Procédé selon la revendication 5, caractérisé en ce que la deshydrogénation selon la deuxième étape du procédé est effectuée par action du nitrite de sodium sur une solution dans l'acide acétique du composé résultant de la première étape dudit procédé.

8. Procédé de désherbage de cultures de coton et tournesol, caractérisé en ce qu'on applique sur les zones cultivées, en prélevée des cultures ou en présemis des cultures avec incorporation dans le sol, une quantité efficace d'une composition selon l'une des revendications 1 à 4.

**Patentansprüche für die Vertragsstaaten BE, CH, DE, FR, GB, IT, LU, NL, SE, LI**

1. Anilinderivat, dadurch gekennzeichnet, dass es der allgemeinen Formel

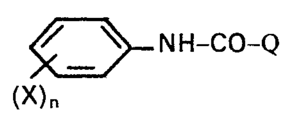

(I)

entspricht, worin

X ein Atom oder einen Rest aus der Gruppe von:

– Halogenatomen,

– Alkylresten mit einem Gehalt von 1 bis 6 Kohlenstoffatomen,

– Alkoxyresten mit einem Gehalt von 1 bis 6 Kohlenstoffatomen,

– Alkenylresten mit einem Gehalt von 2 bis 6 Kohlenstoffatomen,

– Alkenyloxyresten mit einem Gehalt von 2 bis 6 Kohlenstoffatomen,

– Halogenalkylresten mit einem Gehalt von 1 bis 6 Kohlenstoffatomen, die mit einem oder mehreren Halogenatomen substituiert sind,

– Aminorest, gegebenenfalls substituiert mit einem oder zwei gleichen oder verschiedenen Alkylresten, jeweils mit einem Gehalt von 1 bis 6 Kohlenstoffatomen oder mit dem Rest $-CO-R_8$, worin $R_8$ einen Alkylrest mit einem Gehalt von 1

bis 6 Kohlenstoffatomen, einen Alkoxyrest mit einem Gehalt von1 bis 6 Kohlenstoffatomen, einen Alkylaminorest mit einem Gehalt von 1 bis 6 Kohlenstoffatomen oder einen Dialkylaminorest, worin die Alkylreste gleich oder verschieden sein können und 1 bis 6 Kohlenstoffatome enthalten, bedeutet,

– Nitrorest, und

– Cyanrest,

n eine ganze Zahl von 0 bis 5, wobei wenn n grösser als 1 ist, die Substituenten X gleich oder verschieden sein können,

Q einen Rest entsprechend einer der nachstehenden Formeln

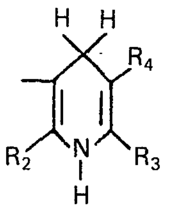

(IA)

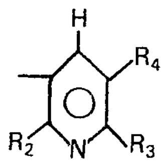

(IB)

worin

– $R_2$ und $R_3$ gleich oder verschieden sind und jeweils ein Wasserstoffatom oder einen Alkylrest mit einem Gehalt von 1 bis 6 Kohlenstoffatomen,

– $R_4$ einen Cyanrest oder einen Rest $-COR_5$, worin $R_5$ eine Hydroxygruppe oder einen Rest $-OR_6$ darstellt, worin $R_6$ einen Alkylrest mit einem Gehalt von 1 bis 6 Kohlenstoffatomen bedeutet, darstellen und die in der Landwirtschaft annehmbaren Salze von diesem Derivat, sowie falls 0 den Rest IA darstellt, die tautomeren Formen von diesem Derivat.

2. Verbindung nach Anspruch 1, dadurch gekennzeichnet, dass

– $R_2$ und $R_3$ gleich oder verschieden sind und ein Wasserstoffatom oder einen Alkylrest mit einem Gehalt von 1 bis 3 Kohlenstoffatomen darstellen,

– $R_4$ einen Alkoxycarbonylrest mit einem Gehalt von 2 bis 6 Kohlenstoffatomen oder den Cyanrest darstellt,

– X ein Atom oder einen Rest aus der Gruppe von Halogenatomen, Alkylresten mit einem Gehalt von 1 bis 4 Kohlenstoffatomen, Alkoxyresten mit einem Gehalt von 1 bis 4 Kohlenstoffatomen, Halogenalkylresten mit einem Gehalt von 1 bis 4 Kohlenstoffatomen und Cyanrest bedeutet,

– n eine ganze Zahl gleich 0, 1, 2, 3 oder 4 darstellt, wobei, falls n grösser als 1 ist, die Substituenten X gleich oder verschieden sein können.

3. Verbindung nach Anspruch 2, dadurch gekennzeichnet, dass sie der Formel

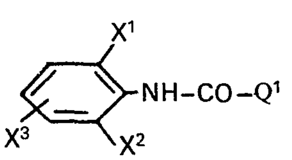

(III)

entspricht, worin

– $X_1$ einen Alkylrest mit einem Gehalt von 1 bis 3 Kohlenstoffatomen,

– $X_2$ ein Wasserstoffatom oder einen Alkylrest mit einem Gehalt von 1 bis 3 Kohlenstoffatomen,

– $X_3$ ein Wasserstoffatom oder ein Halogenatom oder einen Alkylrest mit einem Gehalt von 1 bis 3 Kohlenstoffatomen,

– $Q_1$ einen Rest entsprechend einer der nachstehenden Formeln

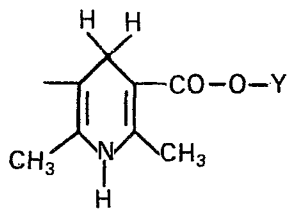

(IIIA)

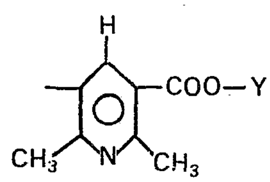

(IIIB)

darstellen, worin

– Y einen Alkylrest mit einem Gehalt von 1 bis 4 Kohlenstoffatomen bedeutet.

4. Herbizide Zusammensetzung, dadurch gekennzeichnet, dass sie als aktives Material wenigstens eine Verbindung gemäss einem der Ansprüche 1 bis 3 enthält.

5. Zusammensetzung nach Anspruch 4, dadurch gekennzeichnet, dass sie 0,001% bis 95%, bezogen auf Gewicht, des aktiven Materials enthält.

6. Verfahren zur Herstellung einer Verbindung nach Anspruch 1, dadurch gekennzeichnet, dass man

(a) ein Anilid der Formel

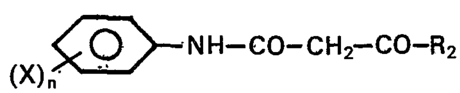

worin X, n und $R_2$ die gleiche Bedeutung wie in Anspruch 1 angegeben besitzen,

(b) Formaldehyd, und

(c) ein Aminoäthylenderivat der Formel

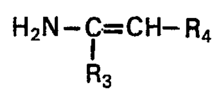

worin $R_3$ und $R_4$ die gleiche Bedeutung wie in Anspruch 1 haben, miteinander zur Umsetzung bringt, um die Verbindung der Formel

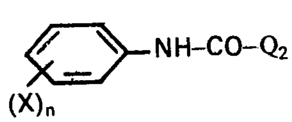

(VII)

zu erhalten, worin X und n die gleiche, vorstehend angegebene Bedeutung besitzen und $Q_2$ den Rest

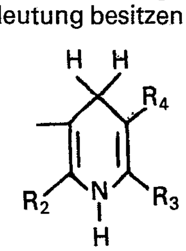

worin $R_2$, $R_3$ und $R_4$ die gleiche Bedeutung wie vorstehend angegeben besitzen, und gegebenenfalls in einer zweiten Stufe die Verbindung der Formel VII in eine Verbindung der nachstehenden Formel

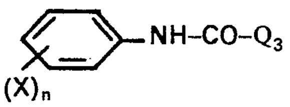

(VIII)

durch Dehydrierung des Restes $Q_2$ umwandelt, worin X und n die gleiche Bedeutung wie vorstehend angegeben besitzen und $Q_3$ den Rest

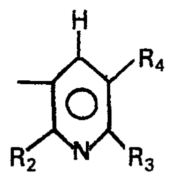

darstellt, worin $R_2$, $R_3$ und $R_4$ die gleiche Bedeutung wie vorstehend angegeben besitzen.

7. Verfahren nach Anspruch 6, dadurch gekennzeichnet, dass die Umsetzung gemäss der ersten Stufe des Verfahrens in Gegenwart eines organischen Lösungsmittels bei einer Temperatur zwischen 15 und 100 °C ausgeführt wird.

8. Verfahren nach Anspruch 6, dadurch gekennzeichnet, dass die Dehydrierung gemäss der zweiten Stufe des Verfahrens durch Einwirkung von Natriumnitrit auf eine Lösung in Essigsäure der in der ersten Stufe des genannten Verfahrens erhaltenen Verbindung bewirkt wird.

9. Verfahren zur Unkrautbekämpfung in Baumwoll- und Sonnenblumenkulturen, dadurch gekennzeichnet, dass man auf die bepflanzten Zonen im Vorauflauf oder vor dem Säen (en présemis) der Kulturen eine wirksame Menge von wenigstens einer Verbindung gemäss einem der Ansprüche 1 bis 3 unter Einverleibung in den Boden aufbringt.

**Patentansprüche für den Vertragsstaat: AT**

1. Herbizide Zusammensetzung, dadurch gekennzeichnet, dass sie als aktives Material wenigstens eine Verbindung entsprechend der Formel I

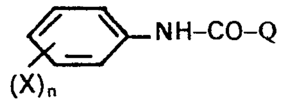

(I)

enthält, worin

X ein Atom oder einen Rest aus der Gruppe von

– Halogenatomen

– Alkylresten mit einem Gehalt von 1 bis 6 Kohlenstoffatomen,

– Alkoxyresten mit einem Gehalt von 1 bis 6 Kohlenstoffatomen,

– Alkenylresten mit einem Gehalt von 2 bis 6 Kohlenstoffatomen,

– Alkenyloxyresten mit einem Gehalt von 2 bis 6 Kohlenstoffatomen,

– Halogenalkylresten mit einem Gehalt von 1 bis 6 Kohlenstoffatomen, die durch ein oder mehrere Halogenatome substituiert sind,

– Aminorest, gegebenenfalls substituiert durch ein oder zwei gleiche oder verschiedene Alkylreste, jeweils mit einem Gehalt von 1 bis 6 Kohlenstoffatomen oder durch den Rest $-CO-R_8$, worin $R_8$ einen Alkylrest mit einem Gehalt von 1 bis 6 Kohlenstoffatomen, einen Alkoxyrest mit einem Gehalt von 1 bis 6 Kohlenstoffatomen, einen Alkylaminorest mit einem Gehalt von 1 bis 6 Kohlenstoffatomen oder einen Dialkylaminorest, worin jeder der Alkylreste, die gleich oder verschieden sind, 1 bis 6 Kohlenstoffatome enthält, bedeutet,

Nitrorest, und

Cyanrest,

n – eine ganze Zahl von 0 bis 5, wobei wenn n grösser als 1 ist, die Substituenten X gleich oder verschieden sein können,

Q – einen Rest entsprechend einer der Formeln IA und IB

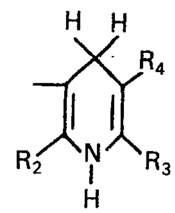

(IA)

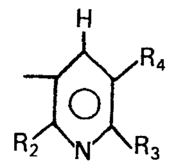

(IB)

worin

– $R_2$ und $R_3$ gleich oder verschieden sind und jeweils ein Wasserstoffatom oder einen Alkylrest mit einem Gehalt von 1 bis 6 Kohlenstoffatomen,

– $R_4$ einen Cyanrest oder einen Rest $-OR_5$, worin $R_5$ einen Hydroxyrest oder einen Rest $-OR_6$ bedeutet, worin $R_6$ einen Alkylrest mit einem Gehalt von 1 bis 6 Kohlenstoffatomen bedeutet, darstellen, oder ein in der Landiwrtschaft annehmbares Salz von diesem Derivat oder wenn Q den Rest IA darstellt, eine tautomere Form von diesem Derivat.

2. Zusammensetzung nach Anspruch 1, dadurch gekennzeichnet, dass sie als aktives Material wenigstens eine Verbindung der Formel I in Anspruch 1 enthält, worin

X ein Atom oder einen Rest aus der Gruppe von

– Halogenatomen,

– Alkylresten mit einem Gehalt von 1 bis 4 Kohlenstoffatomen,

– Alkoxyresten mit einem Gehalt von 1 bis 4 Kohlenstoffatomen,

– Halogenalkylresten mit einem Gehalt von 1 bis 4 Kohlenstoffatomen, und

– Cyanrest,

n eine ganze Zahl, die gleich 0, 1, 2, 3 oder 4 sein kann, wobei, falls n grösser als 1 ist, die Substituenten X gleich oder verschieden sein können,

Q einen Rest entsprechend einer der Formeln IA und IB von Anspruch 1, worin

$-R_2$ und $R_3$, die gleich oder verschieden sind, ein Wasserstoffatom oder einen Alkylrest mit einem Gehalt von 1 bis 3 Kohlenstoffatomen darstellen,

– $R_4$ einen Alkoxycarbonylrest mit einem Gehalt von 2 bis 6 Kohlenstoffatomen oder den Cyanrest darstellt, bedeuten.

3. Zusammensetzung nach Anspruch 2, dadurch gekennzeichnet, dass sie als aktives Material wenigstens eine Verbindung entsprechend der Formel III

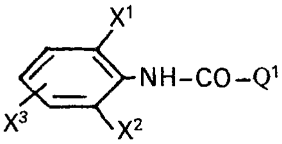

(III)

enthält, worin

– $X_1$ einen Alkylrest mit einem Gehalt von 1 bis 3 Kohlenstoffatomen,

– $X_2$ ein Wasserstoffatom oder einen Alkylrest mit einem Gehalt von 1 bis 3 Kohlenstoffatomen,

– $X_3$ ein Wasserstoffatom oder ein Halogenatom oder einen Alkylrest mit einem Gehalt von 1 bis 3 Kohlenstoffatomen,

– $Q_1$ einen Rest entsprechend einer der nachstehenden Formeln IIIA und IIIB

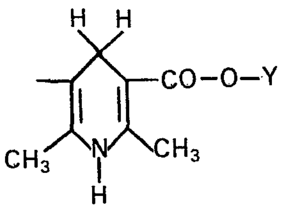

(IIIA)

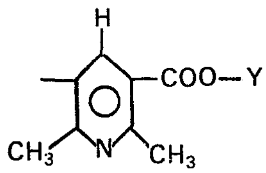

(IIIB)

worin

Y einen Alkylrest mit einem Gehalt von 1 bis 4 Kohlenstoffatomen darstellt, bedeuten.

4. Zusammensetzung nach einem der Ansprüche 1 bis 3, dadurch gekennzeichnet, dass sie 0,001 Gew.-% bis 95 Gew.-% des aktiven Materials enthält.

5. Verfahren zur Herstellung einer Verbindung der Formel I in Anspruch 1, dadurch gekennzeichnet, dass man

(a) ein Anilid der Formel

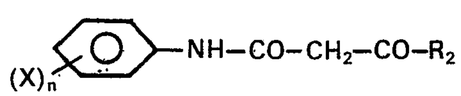

worin X, n und $R_2$ die gleiche Bedeutung wie in Anspruch 1 besitzen,

(b) Formaldehyd, und

(c) ein Aminoäthylenderivat der Formel

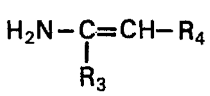

worin $R_3$ und $R_4$ die gleiche Bedeutung wie in Anspruch 1 besitzen,

miteinander zur Umsetzung bringt, um die Verbindung der Formel VII zu ergeben

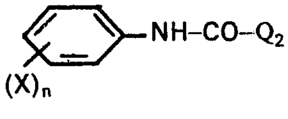

(VII)

worin X und n die gleiche Bedeutung wie vorstehend angegeben besitzen, und $Q_2$ den Rest

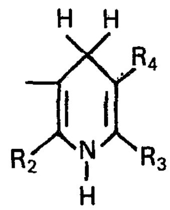

worin $R_2$, $R_3$ und $R_4$ die gleiche Bedeutung wie vorstehend angegeben besitzen, darstellt, und dass man gegebenenfalls in einer zweiten Stufe in Verbindung der Formel VII in eine Verbindung der Formel VIII

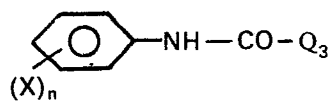

(VIII)

durch Dehydrierung des Restes $Q_2$ überführt, worin X und n die gleiche Bedeutung wie vorstehend angegeben besitzen, und $Q_3$ den Rest

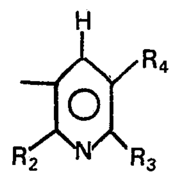

darstellt, worin $R_2$, $R_3$ und $R_4$ die gleiche Bedeutung wie vorstehend angegeben besitzen.

6. Verfahren nach Anspruch 5, dadurch gekennzeichnet, dass man die Umsetzung gemäss der ersten Stufe des Verfahrens in Gegenwart eines organischen Lösungsmittels bei einer Temperatur zwischen 15 und 100 °C ausführt.

7. Verfahren nach Anspruch 5, dadurch gekennzeichnet, dass man die Dehydrierung gemäss der zweiten Stufe des Verfahrens durch Einwirkung von Natriumnitrit auf eine Essigsäurelösung der Verbindung, welche sich nach der ersten Stufe dieses Verfahrens ergibt, ausführt.

8. Verfahren zur Unkrautbekämpfung in Baumwoll- und Sonnenblumenkulturen, dadurch gekennzeichnet, dass man auf die bepflanzten Zonen im Vorauflauf der Kulturen oder vor dem Säen (en présemis) der Kulturen eine wirksame Menge einer Zusammensetzung nach einem der Ansprüche 1 bis 4 unter Einverleibung in den Boden aufbringt.

## Claims for the Contracting States: BE, CH, DE, FR, GB, IT, LI, LU, NL, SE

1. Aniline derivative, characterised in that it corresponds to the general formula:

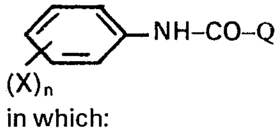

(I)

in which:

– X represents an atom or radical chosen from amongst:

– halogen atoms,

– alkyl radicals containing from 1 to 6 carbon atoms,

– alkoxy radicals containing from 1 to 6 carbon atoms,

– alkenyl radicals containing from 2 to 6 carbon atoms,

– alkenyloxy radicals containing from 2 to 6 carbon atoms,

– halogenalkyl radicals containing from 1 to 6 carbon atoms and substituted by one or more halogens,

– the amino radical optionally substituted by one or two identical or different alkyl radicals each containing from 1 to 6 carbon atoms, or by the radical $-CO-R_8$, in which $R_8$ represents an alkyl radical containing from 1 to 6 carbon atoms, an alkoxy radical containing from 1 to 6 carbon atoms, an alkylamino radical containing from 1 to 6 carbon atoms or a dialkylamino radical in which each of the alkyl parts, which are identical or different, contains from 1 to 6 carbon atoms,

– the nitro radical and

– the cyano radical,

– n is an integer from 0 to 5, it being understood that if n is greater than 1, the substituents X can be identical or different, and

– Q represents a radical corresponding to one of the formulae below:

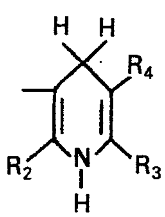

(IA)

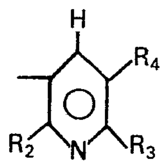

(IB)

in which:

– $R_2$ and $R_3$, which are identical or different, each represent a hydrogen atom or an alkyl radical containing from 1 to 6 carbon atoms, and

– $R_4$ represents the cyano radical or a radical $-COR_5$, in which $R_5$ represents a hydroxyl or a radical $-OR_6$, in which $R_6$ represents an alkyl radical containing from 1 to 6 carbon atoms, and the agriculturally acceptable salts of this derivative and also, if Q represents the radical IA, the tautomeric forms of this derivative.

2. Compound according to Claim 1, characterised in that:

– $R_2$ and $R_4$, which are identical or different, represent the hydrogen atom or an alkyl radical containing from 1 to 3 carbon atoms,

– $R_4$ represents an alkoxycarbonyl radical containing from 2 to 6 carbon atoms or the cyano radical,

– X represents an atom or radical chosen from amongst halogen atoms, alkyl radicals containing from 1 to 4 carbon atoms, alkoxy radicals containing from 1 to 4 carbon atoms, halogenalkyl radicals containing from 1 to 4 carbon atoms and the cyano radical, and

– n is an integer which can be equal to 0, 1, 2, 3 or 4, it being understood that if n is greater than 1, the substituents X can either be identical or different.

3. Compound according to claim 2, characterised in that it corresponds to the formula:

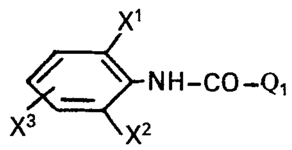

(III)

in which:

– $X_1$ represents an alkyl radical containing from 1 to 3 carbon atoms,

– $X_2$ represents the hydrogen atom or an alkyl radical containing from 1 to 3 carbon atoms,

– $X_3$ represents a hydrogen or halogen atom or an alkyl radical containing from 1 to 3 carbon atoms, and

– $Q_1$ represents a radical corresponding to one of the formulae below:

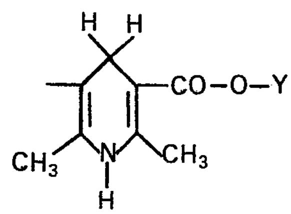

(IIIA)

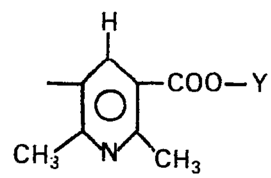

(IIIB)

in which:

– Y represents an alkyl radical containing from 1 to 4 carbon atoms.

4. Herbicidal composition, characterised in that it contains at least one compound according to one of Claims 1 to 3 as the active ingredient.

5. Composition according to Claim 4, characterised in that it contains from 0,001% to 95% weight of active ingredient.

6. Process for the preparation of a compound according to Claim 1, characterised in that the following are reacted together:

a – the anilide of the formula:

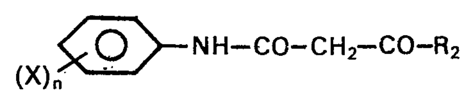

in which X, n and $R_2$ have the same meanings as in Claim 1,

b – formaldehyde and

c – the aminoethylene derivative of the formula:

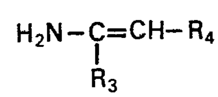

in which $R_3$ and $R_4$ have the same meanings as in Claim 1, to give the compound of the formula (VII):

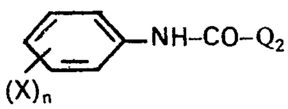

(VII)

in whichX and n have the same meanings as above and $Q_2$ represents the radical:

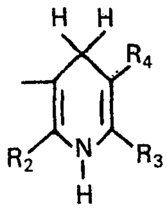

in which $R_2$, $R_3$ and $R_4$ have the same meanings as above, and in that, if appropriate, the compound of the formula (VII) is converted, in a second step, by dehydrogenation of the radical $Q_2$, to a compound of the formula (VIII):

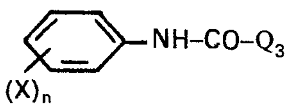

(VIII)

in which X and n have the same meanings as above and $Q_3$ represents the radical:

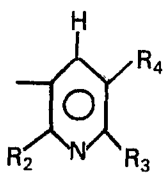

in which $R_2$, $R_3$ and $R_4$ have the same meanings as above.

7. Process according to Claim 6, characterised in that the reaction according to the first step of the process is carried out in an organic solvent medium at a temperature of between 15 °C and 100 °C.

8. Process according to Claim 6, characterised in that the dehydrogenation according to the second step of the process is carried out by reacting sodium nitrite with an acetic acid solution of the compound resulting from the first step of the said process.

9. Process for destroying weeds in cotton and sunflower crops, characterised in that an effective amount of at least one compound according to one of Claims 1 to 3 is applied to the cultivated zones as a pre-emergence treatment of the crops or as a pre-sowing treatment of the crops with incorporation into the soil.

**Claims for the Contracting State: AT**

1. Herbicidal composition, characterised in that it contains, as the active ingredient, at least one compound corresponding to the formula I:

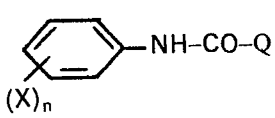

(I)

in which:

– X represents an atom or radical chosen from amongst:

– halogen atoms,

– alkyl radicals containing from 1 to 6 carbon atoms,

– alkoxy radicals containing from 1 to 6 carbon atoms,

– alkenyl radicals containing from 2 to 6 carbon atoms,

– alkenyloxy radicals containing from 2 to 6 carbon atoms,

– halogenoalkyl radicals containing from 1 to 6 carbon atoms and substituted by one or more halogens,

– the amino radical optionally substituted by one or two identical or different alkyl radicals each containing from 1 to 6 carbon atoms, or by the radical $-CO-R_8$, in which $R_8$ represents an alkyl radical containing from 1 to 6 carbon atoms, an alkoxy radical containing from 1 to 6 carbon atoms, an alkylamino radical containing from 1 to 6 carbon atoms or a dialkylamino radical in which each of the alkyl parts, which are identical or different, contains from 1 to 6 carbon atoms,

– the nitro radical and

– the cyano radical,

– n is an integer from 0 to 5, it being understood that if n is greater than 1, the substituents X can be identical or different, and

– Q represents a radical corresponding to one of the formulae IA and IB:

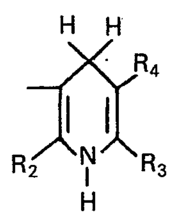

(IA)

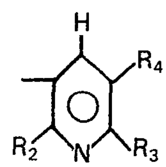

(IB)

in which

– $R_2$ and $R_3$, which are identical or different, each represent a hydrogen atom or an alkyl radical containing from 1 to 6 carbon atoms, and

– $R_4$ represents the cyano radical or a radical $-COR_5$, in which $R_5$ represents a hydroxyl or a radical $-OR_6$, in which $R_6$ represents an alkyl radical containing from 1 to 6 carbon atoms,

or an agriculturally acceptable salt of this derivative or, if Q represents the radical IA, a tautomeric form of this derivative.

2. Composition according to Claim 1, characterised in that it contains, as the active ingredient, at least one compound according to the formula I of Claim 1 in which:

– X represents an atom or radical chosen from amongst halogen atoms, alkyl radicals containing from 1 to 4 carbon atoms, alkoxy radicals containing from 1 to 4 carbon atoms, halogenoalkyl radicals containing from 1 to 4 carbon atoms and the cyano radical,

– n is an integer which can be equal to 0, 1, 2, 3 or 4, it being understood that if n is greater than

1, the substituents X can either be identical or different, and

– Q represents a radical corresponding to one of the formulae IA and IB of Claim 1 in which:

– $R_2$ and $R_3$, which are identical or different, represent the hydrogen atom or an alkyl radical containing from 1 to 3 carbon atoms, and

– $R_4$ represents an alkoxycarbonyl radical containing from 2 to 6 carbon atoms or the cyano radical.

3. Composition according to Claim 2, characterised in that it contains, as the active ingredient, at least one compound corresponding to the formula III:

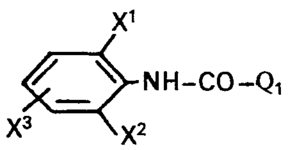

(III)

in which:

– $X_1$ represents an alkyl radical containing from 1 to 3 carbon atoms,

– $X_2$ represents the hydrogen atom or an alkyl radical containing from 1 to 3 carbon atoms,

– $X_3$ represents a hydrogen or halogen atom or an alkyl radical containing from 1 to 3 carbon atoms, and

– $Q_1$ represents a radical corresponding to one of the formulae IIIA and IIIB below:

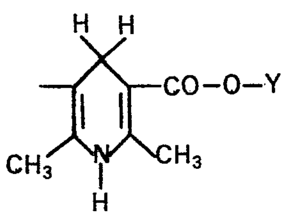

(IIIA)

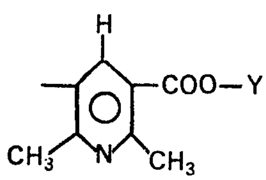

(IIIB)

in which:

– Y represents an alkyl radical containing from 1 to 4 carbon atoms.

4. Composition according to one of Claims 1 to 3, characterised in that it contains from 0,001% to 95% by weight of active ingredient.

5. Process for the preparation of a compound according to the formula I of Claim 1, characterised in that the following are reacted together:

a – the anilide of the formula:

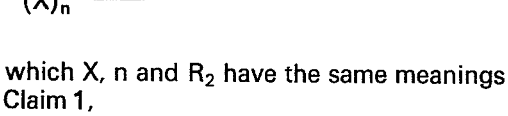

in which X, n and $R_2$ have the same meanings as in Claim 1,

b – bformaldehyde and

c – the aminoethylene derivative of the formula:

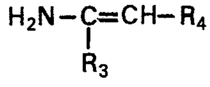

in which $R_3$ and $R_4$ have the same meanings as in Claim 1, to give the compound of the formula (VII):

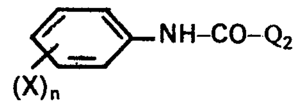

(VII)

in which X and n have the same meanings as above and $Q_2$ represents the radical:

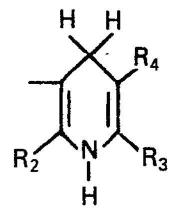

in which $R_2$, $R_3$ and $R_4$ have the same meanings as above, and in that, if appropriate, the compound of the formula (VII) is converted, in a second step, by dehydrogenation of the radical $Q_2$, to a compound of the formula (VIII):

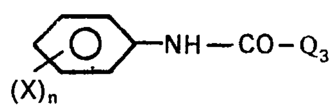

(VIII),

in which X and n have the same meanings as above and $Q_3$ represents the radical:

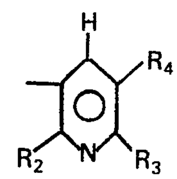

in which $R_2$, $R_3$ and $R_4$ have the same meanings as above.

6. Process according to Claim 5, characterised in that the reaction according to the first step of the process is carried out in an organic solvent medium at a temperature of between 15 °C and 100 °C.

7. Process according to Claim 5, characterised in that the dehydrogenation according to the second step of the process is carried out by reacting sodium nitrite with an acetic acid solution of the compound resulting from the first step of the said process.

8. Process for destroying weeds in cotton and sunflower crops, characterised in that an effective amount of a composition according to one of Claims 1 to 4 is applied to the cultivated zones as a pre-emergence treatment of the crops or as a pre-sowing treatment of the crops with incorporation into the soil.